# FASCICULE DE BREVET EUROPEEN

(11) **EP 0 522 988 B1**
(45) Date de publication et mention de la délivrance du brevet: **02.04.1997**
(21) Numéro de dépôt: 92460021.6
(22) Date de dépôt: 09.07.1992
(51) Int. Cl.: G01N 21/35, G01N 33/18

(54) **Procédé et dispositifs de détection infrarouge de pollution en milieu aqueux**
Verfahren und Vorrichtungen zum Infrarot-Nachweis von Verschmutzungen in wässerigen Medien
Method and arrangements for the infrared detection of pollution in aqueous media

(30) Priorité: 10.07.1991 FR 9108922
(43) Date de publication de la demande: 13.01.1993
(73) Titulaire: GIE ANJOU-RECHERCHE, F-78600 Maisons Laffitte (FR)
(72) Inventeur: Gibert, Michel, F-75009 Paris (FR); Duchevet, Nathalie, F-78600 Maisons Laffitte (FR); Brac, Arnaud, F-75018 Paris (FR); Marin, Guy, F-95390 Saint Prix (FR)
(74) Mandataire: Corlau, Vincent

(56) Documents cités:
- DE-A- 2 923 956
- US-A- 4 207 450
- INTERNATIONAL JOURNAL OF ENVIRONMENTAL ANALYTICAL CHEMISTRY vol. 22, 1 Décembre 1985, pages 109 - 114 A.LIBERATORI ET AL. 'SELECTIVE DETERMINATION OF ORGANIC AND INORGANIC THIOCYANATES ETC.'
- APPLIED SPECTROSCOPY vol. 42, no. 2, 1 Février 1988, pages 228 - 236 M.FULLER ET AL. 'PARTIALLEAST SQUARES QUANTITATIVE ANALYSIS ETC.'
- ANALYTICAL CHEMISTRY vol. 61, no. 8, 15 Avril 1989, pages 898 - 904 D.HOFFMANN ET AL. 'IMPROVED DISCRIMAINATION OF NOISY SPECTRA ETC.'
- APPLIED SPECTROSCOPY vol. 36, no. 3, 1 Juin 1982, pages 223 - 227 C.MANN ET AL. 'SPECTROPHOTOMETRIC ANALYSIS BY CROSS-CORRELATION'

## Description

L'invention concerne le domaine de la détection de la pollution en milieu aqueux, telle que par exemple la détection de la pollution des cours d'eau ou des rejets.

Plus précisément l'invention concerne un procédé et un dispositif de détection de la pollution en milieu aqueux par des composés organiques tels que les hydrocarbures, les organochlorés et les composés aromatiques.

Il existe de nombreuses techniques d'évaluation et/ou d'analyse de la pollution en milieu aqueux. Dans le cadre de la pollution en milieu aqueux par hydrocarbures, la principale méthode utilisée est la spectrophotométrie infrarouge. En effet les hydrocarbures ont la particularité , lorsqu'ils se présentent sous forme de corps purs, de quasiment tous posséder des liaisons dont les vibrations sont à l'origine de l'absorption de radiations électromagnétiques infrarouges possédant une longueur d'onde caractéristique de ladite liaison. Ces molécules possèdent donc une "signature infrarouge" qui se caractérise par l'apparition sur les spectres infrarouges de pics d'absorption de certains faisceaux de radiations possédant une longueur d'onde caractéristique de certaines liaisons ou de certains radicaux présents dans ladite molécule. Les dispositifs d'intégration dont sont munis de nombreux spectrophotomètres permettent de plus de traiter mathématiquement les pics d'absorption et d'obtenir pour chacun d'entre eux une valeur proportionnelle à la concentration des composés organiques détectés.

La spectrophotométrie infrarouge est donc une méthode couramment utilisée permettant de détecter la présence de nombreux hydrocarbures dans un milieu aqueux et dans une certaine mesure de déterminer leur concentration dans ce milieu.

Cependant, cette méthode est peu ou pas utilisée pour détecter les pollutions dues à d'autres composés organiques tels que, par exemple, les composés organochlorés ou les composés aromatiques, malgré le fait que nombre de ces autres composés organiques présentent également une signature infrarouge. Par exemple, la présence d'un radical aromatique se traduira par l'apparition sur le spectre IR d'un pic à 1 600 cm⁻¹ environ. De même, l'existence d'un radical éther se traduira par la présence sur le spectre d'un pic d'absorption situé à environ 1100cm⁻¹.

Dans le domaine de l'analyse infrarouge de la pollution en milieu aqueux, les molécules doivent pouvoir "s'exprimer" librement et par conséquent ne pas être gênées par la présence d'autres matières absorbant dans la région infrarouge du spectre lumineux. L'eau, qui absorbe de façon importante les radiations infrarouges, doit donc pouvoir être écartée de l'échantillon à analyser de façon à obtenir un spectre laissant apparaître les pics d'absorption caractéristiques des composés organiques qui y sont contenus. Dans ce but on utilise des solvants organiques non miscibles à l'eau permettant d'extraire les matières organiques de l'eau à analyser , cette extraction se traduisant par une concentration des composés organiques dans le solvant.

Une contrainte liée au solvant utilisé réside dans le fait qu'il doit absorber le minimum de radiations infrarouges et présenter un spectre infrarouge présentant au moins une fenêtre, c'est-à-dire une zone du spectre au niveau de laquelle le solvant absorbe suffisamment peu, apte à laisser apparaître les pics d'absorption caractéristiques des matières que l'on veut détecter. Les pics d'absorption dus au solvant sont définis comme le bruit de fond du solvant et l'on recherchera l'utilisation de solvants ayant le plus faible bruit de fond possible.

Un autre problème lié au solvant réside dans le fait que la zone observable située à l'intérieur de ces fenêtres spectrales est relativement réduite. Les techniques utilisées pour mieux examiner les pics situés dans ces fenêtres ont consisté à augmenter la sensibilité des dispositifs en utilisant un grand chemin optique et à forcer le signal optique. De telles méthodes posent cependant de nombreux problèmes liés à la luminosité nécessaire à leur mise en oeuvre. Une autre technique a consisté à utiliser des spectrophomètres à transformée de Fourier présentant une plus grande sensibilité et permettant une meilleure isolation des pics d'absorption.

Les méthodes de détection de pollution - en milieu aqueux utlilisées, consistent toutes à étudier une zone restreinte du spectre correspondant à une fenêtre au niveau de laquelle il est possible d'observer les pics d'absorption caractéristiques de certains types de liaison ou de radicaux. Ainsi, les techniques utilisées pour étudier la pollution en milieu aqueux par des hydrocarbures se limitent à étudier la zone du spectre infrarouge au niveau de laquelle on peut distinguer les pics caractéristiques des liaisons les plus couramment rencontrées dans les hydrocarbures. Ainsi la demande de brevet française n° 76 15559 décrit une technique mettant en oeuvre une méthode normalisée de dosage des hydrocarbures dans une phase liquide aqueuse, consistant à rassembler la totalité des hydrocarbures contenu dans une phase aqueuse dans du tétrachlorure de carbone et, après décantation, à mesurer à l'aide d'un spectrophotomètre l'absorption d'un faisceau de radiations infrarouges ayant une longueur d'onde de 3420 nm.

Un des objectifs de la présente invention est de proposer un procédé permettant de détecter non seulement la présence d'hydrocarbures absorbant dans une zone précise du spectre infrarouge mais aussi la présence d'autres composés organiques absorbant en dehors de cette zone, tels que les organochlorés, les composés aromatiques et en particulier de nombreux solvants (xylène, toluène, etc...) les téréphtalates, etc...

Un autre objectif de l'invention est de proposer un procédé permettant de détecter une large gamme de pollutions pouvant apparaître dans un milieu aqueux prédéterminé telle qu'une eau brute. Le procédé selon l'invention peut ainsi être appliqué notamment à la détection de la pollution des eaux de rivière ou des rejets.

Un autre objectif de l'invention est de lutter efficacement contre l'effet "aveuglant" des bruits de fond des solvants utilisés.

Selon l'invention, le procédé de détection de pollution dans un milieu aqueux prédéterminé telle qu'une source d'eau brute est du type comprenant les étapes consistant à :
- extraire les composés organiques contenus dans l'eau à analyser grâce à un solvant absorbant peu ou n'absorbant pas les radiations électromagnétiques infrarouges ;
- utiliser les moyens de spectographie permettant d'obtenir le spectre d'absorption infrarouge desdits composés organiques de ladite eau à analyser.

Le procédé est caractérisé en ce qu'il comprend les étapes consistant à :
- saisir et stocker le spectre infrarouge de référence dudit milieu aqueux prédéterminé, correspondant à la présence de polluants organiques dans cette eau à un taux normal;
- saisir le spectre courant dudit milieu aqueux prédéterminé ; ledit spectre courant et ledit spectre de référence s'étendant au moins de la région allant de 4000 cm⁻¹ environ à 600 cm⁻¹ environ,
- calculer un indice de corrélation dudit spectre courant avec ledit spectre de référence,
- détecter les anomalies.

D'une façon préférentielle, le procédé comprend une étape supplémentaire consistant à déclencher des moyens d'alerte lorsque ledit indice de corrélation franchit une valeur de seuil de corrélation prédéterminé.

Egalement préférentiellement, le spectre courant et le spectre de référence sont obtenus en extrayant les composés organiques du milieu aqueux prédéterminé dans un premier et dans au moins un second solvant absorbant peu les radiations infrarouges, les bandes d'absorption du premier solvant étant distinctes des bandes d'absorption du ou des seconds solvants, lesdits spectre de référence et spectre courant étant chacun obtenu par recoupement des spectres obtenus avec le premier et le ou les seconds solvants.

Avantageusement, lesdits spectre de référence et spectre courant sont obtenus après élimination du bruit de fond du ou des solvants.

Une variante intéressante du procédé consiste à identifier et/ou à quantifier les matières organiques responsables de la pollution.

Selon une variante intéressante, le procédé comprend l'étape complémentaire consistant à éliminer du spectre courant le bruit de fond du milieu aqueux prédéterminé.

Ainsi, le procédé selon l'invention comprend préférentiellement les étapes complémentaires consistant à :
- saisir et stocker les spectres caractéristiques des différents polluants,
- comparer le spectre courant et le spectre de référence de façon à identifier les pics correspondants à des produits de pollution,
- affecter les pics correspondants à des produits de pollution aux spectres stockés caractéristiques de polluants.

Le bruit de fond du milieu aqueux prédéterminé correspond à l'ensemble des pics présents sur le spectre de référence et traduisant la présence en quantité normale de certains composés organiques dans ledit milieux aqueux prédéterminé. Le bruit de fond varie donc en fonction du milieu aqueux considéré.

Avantageusement, le procédé comprend une étape complémentaire consistant à déterminer la concentration des polluants identifiés sur le spectre courant, par exemple par intégration mathématique de la surface des pics.

D'une façon préférentielle, le calcul de l'indice de corrélation du spectre courant avec le spectre de référence, et l'affectation des pics correspondants à des produits de pollution sont effectués en utilisant une méthode mathématique mettant en oeuvre une transformée de Fourier.

L'invention concerne également un dispositif de détection de pollution dans un milieu aqueux prédéterminé comme défini dans la revendication 9.

Enfin, l'invention concerne une station automatique de détection de pollution d'un milieu aqueux prédéterminé. La station automatique selon l'invention est caractérisée en ce qu'elle comprend:
- des moyens de prélèvement d'échantillons dudit milieu aqueux prédéterminé ;
- un dispositif de détection selon l'invention ;
- des moyens d'alarme et/ou de commande pilotés par lesdits moyens de détection permettant de signaler les anomalies.

La station automatique selon l'invention peut être utilisée pour la surveillance de la pollution des eaux brutes tels que les eaux de rivières par prélèvements d'échantillons successifs effectués selon une fréquence déterminée par le risque estimé de pollution. Ainsi, pour la détection de la pollution d'une eau de rivière régulièrement chargée d'effluents industriels les prélèvements et les mesures pourront être très fréquents.

Avantageusement, la station automatique selon l'invention comprend des moyens interactifs permettant d'alimenter une bibliothèque de stockage des spectres de référence de différents milieux aqueux prédéterminés et de différents polluants caractéristiques.

L'invention ainsi que les différents avantages qu'elle présente seront plus facilement compris grâce à l'exemple non limitatif de réalisation qui va suivre en référence aux dessins dans lesquels :
- la figure 1 représente un dispositif de détection de pollution dans un milieu aqueux prédéterminé pour la mise en oeuvre du procédé selon l'invention ;
- la figure 2 représente le spectre infrarouge étendu du tétrachlorure de carbone;
- la figure 3 représente un spectre infrarouge étendu du bromoforme;
- la figure 4 représente le spectre infrarouge étendu de référence de l'eau de Seine ;
- la figure 5 représente le spectre étendu d'un échantillon de la même eau de Seine polluée avec du diméthylphtalate (spectre courant).

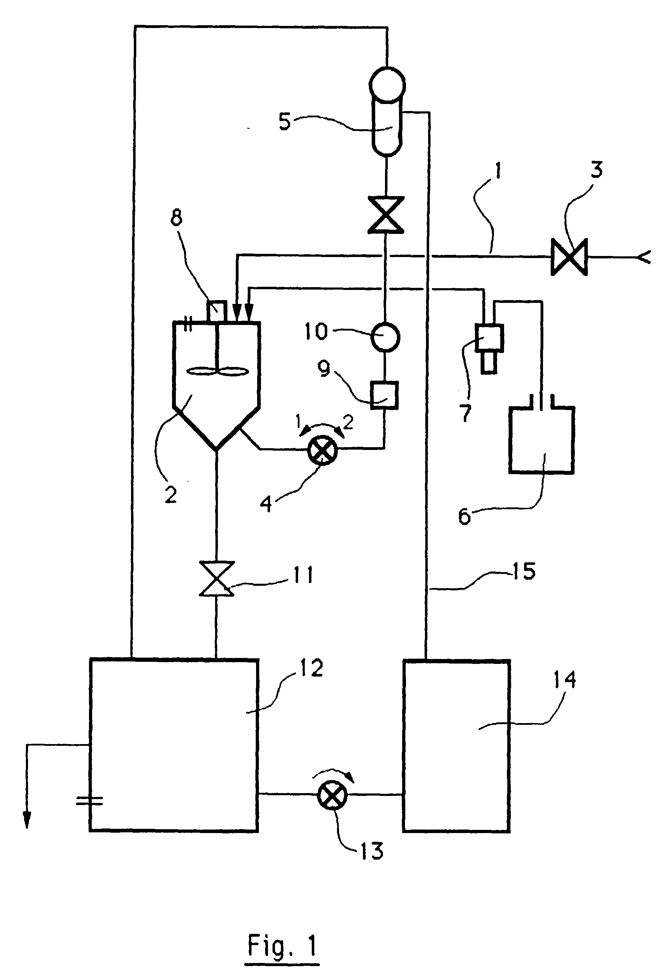

En référence à la figure 1, le dispositif de détection de pollution dans un milieu aqueux prédéterminé pour la mise en oeuvre du procédé selon l'invention comprend :
- des moyens pour extraire les matières organiques contenus dans l'eau à analyser grâce à un solvant absorbant peu ou n'absorbant pas les radiations électromagnétiques infrarouges.
- des moyens de spectographies permettant d'obtenir le spectre d'absorption infrarouge des polluants organiques de l'eau à analyser.

Selon la figure 1, l'eau à analyser est amenée à un conduit 1 jusqu'à un extracteur 2 grâce à une électrovanne 3. Une pompe de transfert 4 permet l'alimentation d'un premier solvant, le tétrachlorure de carbone, d'un réservoir 5 vers l'extracteur 2. L'extraction des matières organiques contenues dans l'eau brute de référence par le tétrachlorure de carbone est favorisée par l'action d'un acide contenu dans un réservoir 6 et amené par une pompe 7 à l'extracteur 2 et par ailleurs par l'action d'une turbine. Une fois l'opération d'extraction terminée, une quantité très précise prédéterminée de solvant chargé de matières organiques est prélevée dans l'extracteur 2 en inversant le sens de fonctionnement de la pompe de transfert 4. Cette quantité de solvant est acheminée jusqu'à une cellule de mesure d'un spectrophotomètre infrarouge 9 (1600 FTIR PERKIN ELMER) qui permet d'obtenir le spectre étendu de ladite eau brute dans le tétrachlorure de carbone. Une cellule 10 contrôle le niveau de remplissage de la cellule de mesure du spectrophotomètre 9. Le temps minimum pour effectuer une mesure est de l'ordre de 10mn et convient aux besoins de la pratique. Lorsque le spectre infrarouge étendu est obtenu, le mélange présent dans l'extracteur 2 est évacué grâce à une pneumovanne 11 vers un décanteur 12 où la séparation entre l'eau et le solvant peut avoir lieu. Bien que le solvant soit utilisé en petite quantité de façon à permettre une concentration rendant détectable la présence des matières organiques qui y sont solubilisées, celui-ci n'est pas rejeté mais recyclé. A cet effet, une conduite 13 permet d'acheminer le solvant récupéré vers un purificateur 14 contenant du charbon actif permettant de séparer le solvant utilisé des matières qui y sont incluses. Ce solvant recyclé est réacheminé vers le réservoir de solvant 5 par une conduite 15.

Le dispositif selon l'invention est par ailleurs muni de moyens (non représentés) permettant de vider entièrement les différents circuits de prélèvement entre deux mesures. Ceci évite tout mélange entre les échantillons successifs et contribue à obtenir la précision voulue des mesures effectuées.

Cette eau supposée polluée subit ensuite le même traitement que l'eau brute de référence. En particulier la quantité de solvant sur laquelle est effectuée la mesure est rigoureusement identique à la quantité de solvant analysée pour l'échantillon de référence. Ce traitement conduit à l'élaboration du spectre infrarouge étendu de l'eau supposée polluée.

Un dispositif identique à celui représenté à la figure 1 permet parallèlement d'obtenir les spectres étendus infrarouges de l'eau brute de référence et de l'eau supposée polluée dans un solvant présentant des bandes d'absorption infrarouges différentes des bandes d'absorption du tétrachlorure de carbone : le bromoforme.

Le spectrophotomètre 9 est équipé d'un moyen de corrélation mathématique par transformée de Fourier permettant d'effectuer une corrélation efficace entre les spectres de référence et les spectres courants des échantillons étudiés.

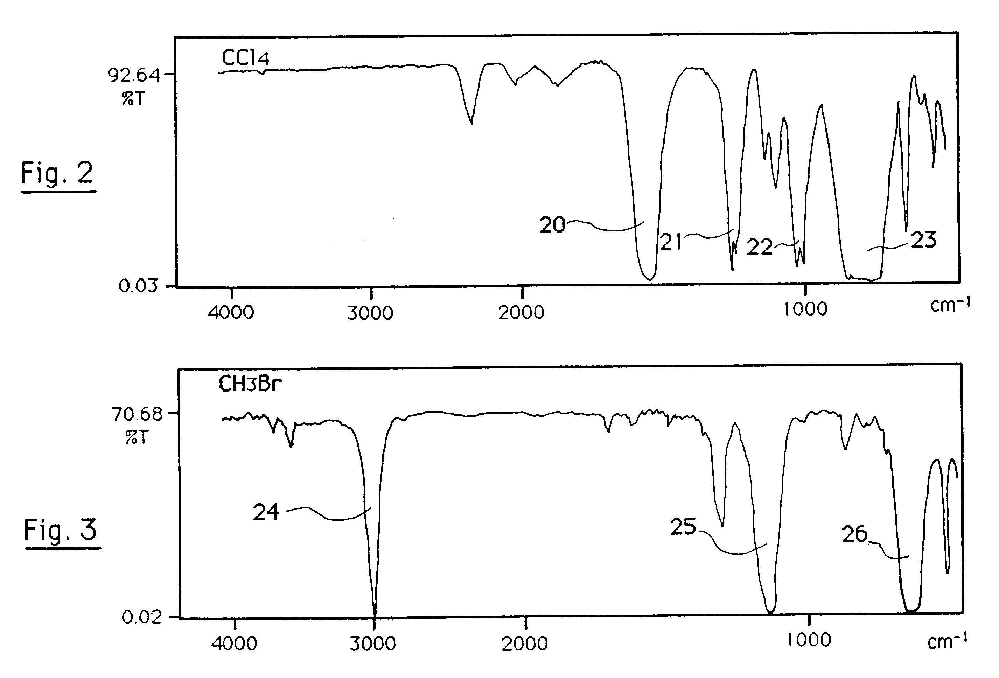

Selon la figure 2, le spectre infrarouge étendu de 4 000 à 600 cm⁻¹ du tétrachlorure de carbone présente différents pics d'absoption 20, 21, 22, 23.

Selon la figure 3, le spectre infrarouge étendu de 4 000 à 600 cm⁻¹ du bromoforme présente également des pics d'absorption 24, 25, 26.

Les pics 20, 21, 22, 23 présentent des valeurs d'absorption différentes des pics 24, 25, 26.

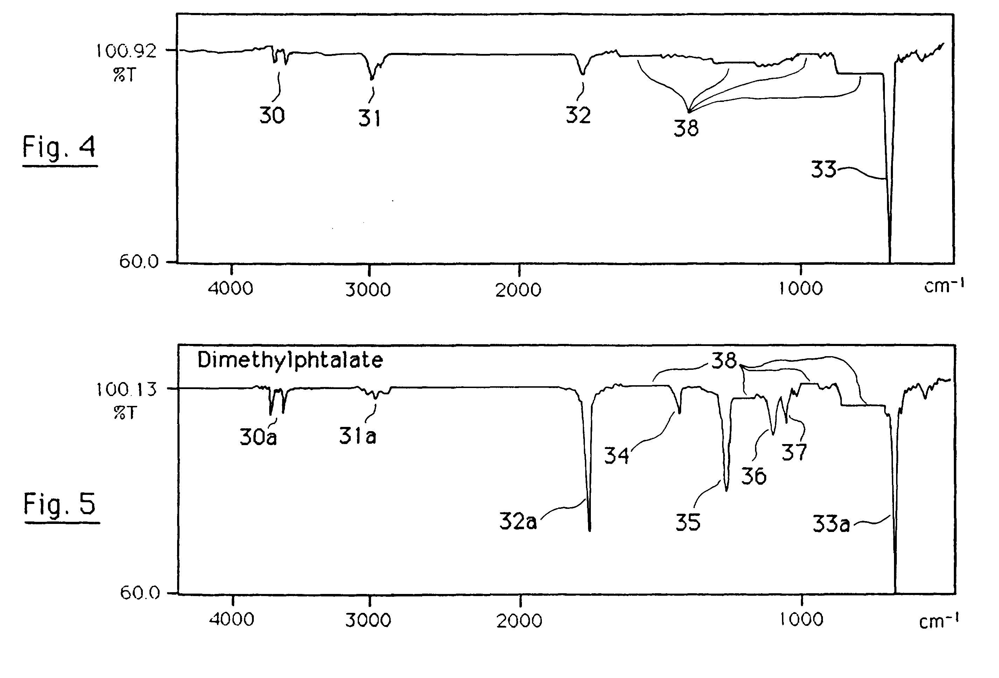

Selon la figure 4, le spectre étendu de l'eau de Seine est obtenu en superposant le spectre étendu de l'eau de Seine extrait par le tétrachlorure de carbone et le spectre infrarouge étendu de l'eau de Seine extrait par le bromoforme. Le bruit de fond du solvant correspondant à des zones non observables et se traduisant sur les spectres des solvants par les pics d'absorption 20 à 26 a été éliminé de façon à visualiser plus facilement les différents pics caractéristiques de l'eau de Seine. L'élimination du bruit de fond du solvant se traduit par la présence de zones aveugles 38. Ce spectre présente par ailleurs des pics d'absorption 31, 32, 33, correspondant à la présence de polluants organiques dans cette eau à un taux normal.

Selon la figure 5, le spectre représenté correspond au spectre infrarouge étendu d'une eau de Seine polluée. Les pics 30, 31, 32, 33, représentatifs de l'eau de Seine de référence se retrouvent sur ce spectre sous la forme des pics 30a, 31a, 32a, 33a. La présence d'autres pics d'absorption 34, 35, 36, 37, traduit l'existence d'une pollution par un autre corps que les hydrocarbures contenus dans l'eau de Seine de référence.

La présence des pics 34, 35, 36, 37 ainsi que le dépassement du seuil du pic d'absorption 31a provoque la délivrance de signaux qui sont utilisés pour commander une alarme significative d'une pollution.

La nature précise de la pollution peut ensuite être analysée de façon précise grâce à une bibliothèque de spectres gérée par un logiciel SADTLER IR search software. Les valeurs d'absorption moyennes des pics 34, 35, 36, 37 permettent d'identifier le produit polluant comme étant du diméthylphtalate. L'intégration de la courbe suivie par ces différents pics permet par ailleurs de quantifier la présence de ce produit et révèle une concentration de 2mg/l.

## Revendications

1. Procédé de détection de pollution dans un milieu aqueux prédéterminé tel qu'une source d'eau brute ou de rejet du type comprenant les étapes consistant à :
- extraire les composés organiques contenus dans l'eau à analyser grâce à un solvant absorbant peu ou n'absorbant pas les radiations électromagnétiques infrarouges ;
- utiliser les moyens de spectographie permettant d'obtenir le spectre d'absorption infrarouge desdits composés organiques de ladite eau à analyser dans ledit solvant ;
caractérisé en ce qu'il comprend les étapes consistant à :
- saisir et stocker le spectre infrarouge de référence dudit milieu aqueux prédéterminé, correspondant à la présence de polluants organiques dans cette eau à un taux normal;
- saisir le spectre courant dudit milieu aqueux prédéterminé ; ledit spectre courant et ledit spectre de référence s'étendant au moins de la région allant de 4000 cm⁻¹ à 600 cm⁻¹ environ,
- calculer un indice de corrélation dudit spectre courant avec ledit spectre de référence,
- détecter les anomalies.

2. Procédé de détection de pollution selon la revendication 1 caractérisé en ce qu'il comprend l'étape supplémentaire consistant à déclencher des moyens d'alerte lorsque ledit indice de corrélation franchit une valeur de seuil prédéterminée.

3. Procédé de détection selon l'une des revendications 1 ou 2 caractérisé en ce que le spectre courant et le spectre de référence sont obtenus en extrayant les composés organiques du milieu aqueux prédéterminé dans un premier et dans au moins un second solvant absorbant peu les radiations infrarouges, les bandes d'absorption du premier solvant étant distinctes des bandes d'absorption du ou des seconds solvants, lesdits spectre de référence et spectre courant étant chacun obtenu par recoupement des spectres obtenus avec le premier et le ou les seconds solvants.

4. Procédé de détection de pollution selon l'une des revendications 1 à 3 caractérisé en ce que lesdits spectre de référence et spectre courant sont obtenus après élimination du bruit de fond du ou des solvants.

5. Procédé de détection de pollution selon l'une des revendications 1 à 4 caractérisé en ce qu'il comprend l'étape complémentaire consistant à éliminer du spectre courant le bruit de fond du milieu aqueux prédéterminé.

6. Procédé de détection de pollution selon l'une des revendications 1 à 5 caractérisé en ce qu'il comprend les étapes complémentaires consistant à :
- saisir et stocker les spectres caractéristiques des différents polluants,
- comparer le spectre courant et le spectre de référence de façon à identifier les pics correspondants à des produits de pollution,
- affecter les pics correspondants à des produits de pollution aux spectres stockés caractéristiques de polluants.

7. Procédé de détection de pollution selon la revendication 6 caractérisé en ce qu'il comprend une étape complémentaire consistant à déterminer la concentration des polluants identifiés sur le spectre courant.

8. Procédé de détection de pollution selon l'une des revendications 1 à 7 caractérisé en ce que le calcul de l'indice de corrélation du spectre courant avec le spectre de référence, et l'affectation des pics correspondants à des produits de pollution sont effectués en utilisant une méthode mathématique mettant en oeuvre une transformée de Fourier.

9. Dispositif de détection de pollution dans un milieu aqueux prédéterminé pour la mise en oeuvre du procédé selon l'une des revendications 1 à 8, comprenant :
- un moyen pour extraire les composés organiques contenus dans l'eau à analyser grâce à un solvant absorbant peu ou n'absorbant pas les radiations électromagnétiques infrarouges ;
- un spectrophotomètre infrarouge permettant d'obtenir le spectre d'absorption infrarouge desdits composés organiques, qui s'etend au moins de la région allant de 4000 cm⁻¹ à 600 cm⁻¹ environ, incluant un moyen de stockage contenant le spectre de référence correspondant à la présence de polluants organiques dans cette eau à un taux normal et incluant un moyen de corrélation mathématiques, qui permet de calculer un indice de corrélation d'un spectre courant avec ledit spectre de référence.

10. Station automatique de détection de pollution d'un milieu aqueux prédéterminé caractérisé en ce qu'elle comprend :
- des moyens de prélèvement d'échantillons dudit milieu aqueux prédéterminé ;
- un dispositif de détection selon la revendication 9 ; selon
- des moyens d'alarme et/ou de commande pilotés par ledit dispositif de détection permettant de signaler les anomalies.

11. Station automatique selon la revendication 10 caractérisé en ce qu'elle comprend des moyens interactifs permettant d'alimenter une bibliothèque de stockage des spectres de référence de différents milieux aqueux prédéterminés et de différents polluants caractéristiques.

## Patentansprüche

1. Verfahren zum Nachweis der Verschmutzung in einer vorgegebenen wässrigen Umgebung wie einer schmutzigen Wasserquelle oder Abwasser, das die folgenden Schritte umfaßt:
- es werden die im zu analysierenden Wasser enthaltenen organischen Verbindungen mittels eines Lösungsmittels extrahiert, welches die elektromagnetische Infrarot-Strahlung gering oder nicht absorbiert;
- es werden diejenigen Mittelwerte aus der Spektrographie verwendet, die es erlauben, ein Infrarot-Absorptionsspektrum der organischen Verbindungen des zu analysierenden Wassers in dem Lösungsmittel zu erhalten;
dadurch gekennzeichnet, daß die folgenden Schritte umfaßt sind:
- es wird das Referenz-lnfrarotspektrum der vorgegebenen wässrigen Umgebung aufgenommen und gespeichert, entsprechend der Anwesenheit von organischen Schadstoffen in diesem Wasser in normaler Konzentration;
- es wird das momentane Spektrum der vorgegebenen wässrigen Umgebung aufgenommen, wobei sich das momentane Spektrum und das Referenzspektrum mindestens über den Bereich von ungefähr 4000 cm⁻¹ bis 600 cm⁻¹ erstrecken,
- es wird ein Korrelationsindex des momentanen Spektrums mit Hilfe des Referenzspektrums ausgerechnet,
- es werden die Anomalien detektiert.

2. Verfahren nach Anspruch 1,
dadurch gekennzeichnet, daß als zusätzlicher Schritt Warnmittel ausgelöst werden, wenn der Korrelationsindex einen vorgegebenen Schwellenwert überschreitet.

3. Verfahren nach Anspruch 1 oder 2,
dadurch gekennzeichnet, daß das momentane Spektrum und das Referenzspektrum durch Extraktion der organischen Verbindungen in der vorgegebenen wässrigen Umgebung in einem ersten und mindestens in einem zweiten Lösungsmittel erhalten werden, welches die Infrarotstrahlung gering absorbiert, wobei die Absorptionsbanden des ersten Lösungsmittels von den Absorptionsbanden des oder der zweiten Lösungsmittel verschieden sind, wobei das Referenzspektrum und das momentane Spektrum jeweils durch Vergleich der mit dem ersten und dem oder den zweiten Lösungsmitteln erhaltenen Spektren erhalten werden.

4. Verfahren nach einem der Ansprüche 1 bis 3,
dadurch gekennzeichnet, daß das Referenzspektrum und das momentane Spektrum nach Elimination des Hintergrundrauschens des oder der Lösungsmittel erhalten werden.

5. Verfahren nach einem der Ansprüche 1 bis 4,
dadurch gekennzeichnet, daß als zusätzlicher Schritt vom momentanen Spektrum das Hintergrundrauschen der vorgegebenen wässrigen Umgebung eliminiert wird.

6. Verfahren einem der Ansprüche 1 bis 5,
dadurch gekennzeichnet, daß die zusätzlichen Schritte umfaßt werden:
- es werden die charakteristischen Spektren der verschiedenen Schadstoffe aufgenommen und gespeichert;
- es werden das momentane Spektrum und das Referenzspektrum miteinander verglichen, um die Peaks, die Verschmutzungsprodukten entsprechen, zu identifizieren;
- es werden die Peaks, die Verschmutzungsprodukten entsprechen, den aufgenommenen Spektren der charakteristischen Schadstoffe zugeordnet.

7. Verfahren nach Anspruch 6,
dadurch gekennzeichnet, daß als zusätzlicher Schritt die Konzentration der Schadstoffe bestimmt wird, die im momentanen Spektrum identifiziert werden.

8. Verfahren nach einem der Ansprüche 1 bis 7,
dadurch gekennzeichnet, daß die Berechnung des Korrelationsindex des momentanen Spektrums mit Hilfe des Referenzspektrums und die Zuordnung der Peaks, die Verschmutzungsprodukten entsprechen, unter Verwendung einer mathematischen Methode, die eine Fouriertransformation benutzt, durchgeführt werden.

9. Vorrichtung zum Nachweis von Verschmutzung in einer vorgegebenen wässrigen Umgebung zum Durchführen des Verfahrens gemäß einem der Ansprüche 1 bis 8, umfassend:
- ein Mittel zur Extraktion der im zu analysierenden Wasser enthaltenen organischen Verbindungen mittels eines Lösungsmittels, welches die elektromagnetische Infrarotstrahlung gering oder nicht absorbiert;
- ein Infrarotspektrometer, welches es erlaubt, ein sich mindestens über den Bereich von ungefähr 4000 cm⁻¹ bis 600 cm⁻¹ erstreckendes Infrarotabsorptionsspektrum der organischen Verbindungen zu erhalten, einschließlich eines Speichermediums, welches das Referenzspektrum enthält, das der Anwesenheit von organischen Schadstoffen in diesem Wasser in normaler Konzentration entspricht und einschließlich eines mathematischen Korrelationsmittels, welches die Berechnung eines Korrelationsindex eines momentanen Spektrums mit Hilfe des Referenzspektrums erlaubt.

10. Automatische Station zum Nachweis von Verschmutzung in einer vorgegebenen wässrigen Umgebung,
dadurch gekennzeichnet, daß sie umfaßt:
- Mittel zur Probenentnahme aus der vorgegebenen wässrigen Umgebung;
- eine Nachweisvorrichtung gemäß Anspruch 9;
- Alarmmittel und/oder Steuerungsmittel für die Nachweisvorrichtung zur Anzeige der Anomalien.

11. Automatische Station nach Anspruch 10,
dadurch gekennzeichnet, daß sie interaktive Mittel umfaßt, die es erlauben, eine Bibliothek mit gespeicherten Referenzspektren der verschiedenen vorgegebenen wässrigen Umgebungen und der verschiedenen charakteristischen Schadstoffe zu speisen.

## Claims

1. Method for detecting pollution in a predetermined aqueous medium, such as an untreated or waste water source, of the type comprising steps consisting in:
- extracting the organic compounds contained in the water to be analyzed using a solvent which absorbs little or does not absorb infrared electromagnetic radiation;
- using spectrography means making it possible to obtain the infrared absorption spectrum of the said organic compounds of the said water to be analyzed in the said solvent;
characterized in that it comprises the steps consisting in:
- recording and storing the reference infrared spectrum of the said predetermined aqueous medium, corresponding to the presence of organic pollutants in this water at a normal level;
- recording the current spectrum of the said predetermined aqueous medium;
the said current spectrum and the said reference spectrum extending at least in the range from about 4000 cm⁻¹ to 600 cm⁻¹,
- calculating a correlation index of the said current spectrum with the said reference spectrum,
- detecting anomalies.

2. Method for detecting pollution according to Claim 1, characterized in that it comprises the additional step consisting in triggering warning means when the said correlation index exceeds a predetermined threshold value.

3. Detection method according to one of Claims 1 and 2, characterized in that the current spectrum and the reference spectrum are obtained by extracting the organic compounds from the predetermined aqueous medium in a first and in at least a second solvent absorbing little infrared radiation, the absorption bands of the first solvent being distinct from the absorption bands of the second solvent or solvents, the said reference spectrum and current spectrum each being obtained by cross-checking the spectra obtained with the first solvent and the second solvent or solvents.

4. Method for detecting pollution according to one of Claims 1 to 3, characterized in that the said reference spectrum and current spectrum are obtained after eliminating the background noise of the solvent or solvents.

5. Method for detecting pollution according to one of Claims 1 to 4, characterized in that it comprises the complementary step consisting in eliminating the background noise of the predetermined aqueous medium from the current spectrum.

6. Method for detecting pollution according to one of Claims 1 to 5, characterized in that it comprises the complementary steps consisting in:
- recording and storing the characteristic spectra of the various pollutants,
- comparing the current spectrum and the reference spectrum so as to identify the peaks corresponding to pollution products,
- assigning the peaks corresponding to pollution products to stored characteristic spectra of pollutants.

7. Method for detecting pollution according to Claim 6, characterized in that it comprises a complementary step consisting in determining the concentration of the pollutants identified on the current spectrum.

8. Method for detecting pollution according to one of Claims 1 to 7, characterized in that calculation of the correlation index of the current spectrum with the reference spectrum and assignment of the peaks corresponding to pollution products are carried out using a mathematical method employing a Fourier transform.

9. Device for detecting pollution in a predetermined aqueous medium, for implementing the method according to one of Claims 1 to 8, comprising:
- a means for extracting the organic compounds contained in the water to be analyzed using a solvent which absorbs little or does not absorb infrared electromagnetic radiation;
- an infrared spectrophotometer, making it possible to obtain the infrared absorption spectrum of the said organic compounds which extends at least in the range from about 4000 cm⁻¹ to 600 cm⁻¹, including a storage means containing the reference spectrum corresponding to the presence of organic pollutants in this water at a normal level, and including a mathematical correlation means which makes it possible to calculate a correlation index of a current spectrum with the said reference spectrum.

10. Automatic station for detecting pollution of a predetermined aqueous medium, characterized in that it comprises,
- means for taking samples of the said predetermined aqueous medium;
- a detection device according to Claim 9;
- warning and/or control means driven by the said detection device, making it possible to indicate anomalies.

11. Automatic station according to Claim 10, characterized in that it comprises interactive means making it possible to supply a library for storing the reference spectra of various predetermined aqueous media and of various characteristic pollutants.
